# EUROPÄISCHE PATENTSCHRIFT

(11) **EP 3 649 049 B1**
(45) Veröffentlichungstag und Bekanntmachung des Hinweises auf die Patenterteilung: **09.06.2021**
(21) Anmeldenummer: 18737219.8
(22) Anmeldetag: 29.06.2018
(51) Int. Cl.: B65B 3/00, B65B 65/06, B65B 55/12, B65B 55/10, B65B 43/52, B65B 55/02, B65B 43/42, B65B 43/26, B65B 25/00, B65B 43/50, B65B 43/44, B08B 9/42, B65G 17/34, B65G 15/58, A61L 2/20, B65B 59/04, B65B 51/14, B65B 43/54, B65G 65/32

(54) **ZELLENTRAVERSE FÜR EINE FÜLLMASCHINE**
CELL-TYPE CROSSMEMBER FOR A FILLING MACHINE
TRAVERSE DE CELLULE POUR UNE MACHINE DE REMPLISSAGE

(30) Priorität: 03.07.2017 DE 102017114759
(43) Veröffentlichungstag der Anmeldung: 13.05.2020
(73) Patentinhaber: SIG Technology AG, 8212 Neuhausen am Rheinfall (CH)
(72) Erfinder: LÜNNEMANN, Bernd, 40489 Düsseldorf (DE); NABER, Klaus, 47906 Kempen (DE); VETTEN, Thomas, 40223 Düsseldorf (DE); CRAMER, Thomas, 31582 Nienburg (Weser) (DE); SOBOTKA, Peter, 40625 Düsseldorf (DE)
(74) Vertreter: Cohausz & Florack
(86) Internationale Anmeldenummer: PCT/EP2018/067659
(87) Internationale Veröffentlichungsnummer: WO 2019/007851

(56) Entgegenhaltungen:
- DE-A1- 3 701 079
- DE-U1- 20 320 380
- GB-A- 853 696
- US-A1- 2010 263 324
- US-A1- 2013 146 423

## Beschreibung

Die Erfindung betrifft eine Zellentraverse für einen Aseptikbereich einer Füllmaschine zum Füllen von Packungen mit fließfähigen Produkten. Die Erfindung betrifft weiterhin eine Verwendung einer erfindungsgemäßen Zellentraverse und eine Füllmaschine.

Durch gattungsgemäße Füllmaschinen werden Packungen vorzugsweise mit fließfähigen Lebensmitteln gefüllt. Als Packungen werden insbesondere Packungen verwendet, die an einer Oberseite offen sind, um eine Öffnung zum Füllen der Packungen bereitzustellen. Die Packungen können beispielsweise Kartonverbundpackungen sein, die aus einem Packstoff in Form eines Laminats umfassend eine Kartonschicht und äußeren, insbesondere thermoplastischen, Kunststoffschichten, etwa aus Polyethylen (PE), gebildet sind. Der Karton verleiht den Packungen eine ausreichende Stabilität, damit die Packungen einfach gehandhabt und beispielsweise gestapelt werden können. Die Kunststoffschichten schützen den Karton vor Feuchtigkeit und erlauben ein Siegeln des Packstoffs zur Bildung einer dichten Packung. Zusätzlich können noch weitere Schichten, wie etwa eine Aluminiumschicht, vorgesehen sein, die eine Diffusion von Sauerstoff und anderen Gasen durch die Packung verhindern. Eine Packung kann jedoch auch eine Flasche, beispielsweise eine PET-Flasche, sein.

Das Füllen der Packungen mit Lebensmitteln erfolgt typischerweise unter sterilen Bedingungen. Dabei ist nicht nur das abzufüllende Lebensmittel, sondern auch die Packung zu sterilisieren. Dazu werden die Lebensmittel normalerweise für eine bestimmte Zeitspanne erhitzt. Die Packungen werden zunächst meist mit steriler Heißluft ausgeblasen. In die so aufgeheizten Packungen wird dann ein Sterilisierungsmittel eingeleitet, das typischerweise Wasserstoffperoxid ist oder wenigstens aufweist. Da die Packung vorgeheizt ist, werden hohe Reaktionsgeschwindigkeiten bei der Sterilisierung erreicht und vermieden, dass sich Kondensat in der Packung bildet. Feuchtigkeit und restliches Wasserstoffperoxid werden anschließend durch Trocknen der Packung mit, vorzugsweise heißer und steriler, Trocknungsluft aus der Packung entfernt. Sodann erfolgt ebenfalls unter sterilen Bedingungen das Füllen der sterilen Packung mit dem ebenfalls sterilen Produkt, das vorwiegend fließfähig, insbesondere flüssig ist. Entsprechende Produkte sind typischerweise Lebensmittel, wie etwa Säfte, Milch, Soßen und dergleichen. Dabei können die Lebensmittel neben der wenigstens einen flüssigen Komponente auch stückige Anteile aufweisen. Nachdem die Packungen gefüllt sind, werden diese noch unter steriler Atmosphäre verschlossen.

Während des Sterilisierens und/oder des Füllens können die Packungen kontinuierlich, bedarfsweise mit konstanter Geschwindigkeit, durch die Füllmaschine transportiert werden. Alternativ werden die Packungen jedoch taktweise, also schrittweise, durch die Füllmaschine bewegt. Dabei können die Packungen nacheinander jeweils für eine bestimmte Zeitspanne bestimmte Positionen einnehmen, in denen die Packungen stillstehend über wenigstens eine Düse mit Heißluft, Sterilisierungsmittel und/oder Trocknungsluft beaufschlagt bzw. mit dem abzufüllenden Produkt gefüllt werden können.

Dem gezielten Transport der Packungen durch die Füllmaschine dient häufig eine Transporteinrichtung, die eine Reihe von umlaufend bewegbaren Zellentraversen mit Zellen aufweist. Die Zellen einer Zellentraversen durchlaufen typischerweise gleichzeitig den gleichen Verfahrensschritt der Füllmaschine. Durch das Vorsehen mehrere parallel verlaufender Zellentraversen hintereinander entstehen hintereinander angerordnete Zellen. In einem Zuführbereich werden die Packungen von einer Zuführeinrichtung nacheinander in die Zellen der hintereinander angeordneten Zellentraversen übergeben. Die Packungen werden dann in den Zellen durch einen Füllbereich zu einem Abgabebereich bewegt, währenddessen die Packungen wie beschrieben sterilisiert, befüllt und verschlossen werden. Vom Sterilisieren bis zum Verschließen der Packung und insbesondere beim Füllen der Packungen ist ein aseptischer oder keimfreier Bereich (Aseptikbereich) erforderlich. Die Zellentraversen dürfen somit möglichst keine Keime oder andere Verunreinigungen in den Aseptikbereich mit einschleppen. Gleichzeitig müssen die Zellentraversen den Bedingungen im Aseptikbereich (hohe Temperaturen, Wasser, Sterilisierungsmittel wie beispielswiese Wasserstoffperoxid) standhalten können. Im Abgabebereich werden die Packungen dann über eine entsprechende Abgabeeinrichtung abgegeben. Die nun leeren Zellen werden dann über einen Rückführbereich zum Zuführbereich zurückbewegt, um dort wieder zu füllende, insbesondere einseitig offene, Packungen aufnehmen zu können. Typischerweise sind der Füllbereich und der Rückführbereich etwa geradlinig ausgebildet, während dazwischen Umlenkbereiche vorgesehen sind, in den die Transportrichtung der Zellen umgekehrt wird. Die Zuführeinrichtung und die Abgabeeinrichtung sind dabei aus Platzgründen typischerweise den Umlenkbereichen zugeordnet.

Um die Verschmutzung und die Keimfreiheit der Zellentraverse möglichst lange aufrechterhalten zu können, macht man sich zum einen das Kühlwasser zunutze, welches dazu verwendet wird die Transporteinrichtung inklusive der Zellentraverse zu kühlen. Zu diesem Zweck wird die Transporteinrichtung im Rückführbereich mittels Düsen mit Kühlwasser bespritzt. Dadurch wird an den Zellentraversen anhaftender Schmutz wenigstens teilweise entfernt, sodass eine Ansammlung von Schmutz während des Betriebs in vielen Fällen vermieden werden kann.

Allerdings können Spritzer von hochviskosen Produkten, wie beispielsweise Soßen, sehr haftstarke Verschmutzungen der Transporteinrichtung und der Zellentraverse bilden, die vom Kühlwasser der Zellenkühlung nicht effektiv genug entfernt werden. Daher können sich entsprechende Verunreinigungen ansammeln und das Wachstum von Mikroorganismen begünstigen. Dies kann dazu führen, dass das Produkt in den Packungen durch den Eintrag von Mikroorganismen kontaminiert wird. Die Packungen weisen dann eine verminderte Qualität auf, da die abgefüllten Produkte schneller als gewünscht verderben. Deshalb ist es notwendig, die Produktion in relativ kurzen Abständen für eine Zwischen- oder Vollreinigung zu unterbrechen.

Die DE 3701079 A1 offenbart ein Verfahren zum Entkeimen von Verpackungsbehältern, bei dem diese zunächst vorgewärmt werden, darauf ein Entkeimungsmittel zusammen mit Warmluft auf die Behälteroberflächen aufgeblasen wird und schließlich das Entkeimungsmittel wieder entfernt wird. Um das Entkeimungsmittel nach dessen Einwirkung völlig zu beseitigen, werden die Verpackungsbehälter mit ihren Öffnungen nach unten weisend mit Sterilwasser gespült und anschließend mit Sterilluft ausgeblasen.

Alle bisherigen Versuche, der bestehenden Problematik hinreichend Rechnung zu tragen, etwa indem die Zellenkühlung so intensiviert wird, dass die Transporteinrichtung besser gereinigt werden kann reichen nicht aus, um die Produktionszyklen auf die gewünschten Zeiträume ausdehnen zu können. Dies liegt beispielsweise an dem geringen zur Verfügung stehenden Bauraum und/oder daran, dass bei der Reinigung der Transporteinrichtung sekundäre Anlagenteile der Füllmaschine verschmutzt werden und/oder dass es trotzdem zu einem Eintrag von Mikroorganismen in die Packungen kommt.

Daher liegt der vorliegenden Erfindung die Aufgabe zugrunde, eine gattungsgemäße Zellentraverse derart auszugestalten und weiterzubilden, dass die Gefahr der Ansammlung von Verunreinigungen und der Kontamination von abgefülltem Produkt durch Mikroorganismen verringert oder verhindert werden kann.

Gemäß einer ersten Lehre der vorliegenden Erfindung wird die Aufgabe mit einer gattungsgemäßen Zellentraverse gelöst, welche eine Rahmenstruktur mit zumindest einer ersten seitlichen Längsstruktur, mit zumindest einer der ersten Längsstruktur gegenüberliegenden zweiten seitlichen Längsstruktur, und mit mehreren zwischen der ersten und der zweiten Längsstruktur angeordneten und die Längsstrukturen verbindenden Querelementen umfasst, wobei zwischen den Längsstrukturen und den Querelementen mehrere in Längsrichtung der Zellentraverse nebeneinander liegende Zellen zur Aufnahme von zu füllenden oder gefüllten Packungen gebildet werden, dadurch gekennzeichnet, dass die Zellen jeweils durch die Längsstrukturen und Querelemente gebildet werden, wobei entweder die erste Längsstruktur und/oder die zweite Längsstruktur als im Wesentlichen flächige Seitenwand ausgebildet ist und zumindest ein Teil der Querelemente im Wesentlichen rohrförmig ausgebildet ist, oder die erste Längsstruktur und/oder die zweite Längsstruktur jeweils zumindest eine, vorzugsweise zumindest zwei rohrförmige Längsstreben umfasst und zumindest ein Teil der Querelemente im Wesentlichen flächig ausgebildet ist, und wobei zumindest ein Teil der Querelemente ausschließlich formschlüssig und/oder kraftschlüssig mit den Längsstrukturen verbunden ist.

Gemäß einer zweiten Lehre wird die Aufgabe auch durch eine Verwendung einer erfindungsgemäßen Zellentraverse für eine Füllmaschine zum Füllen von Packungen mit fließfähigen Produkten gelöst.

Gemäß einer dritten Lehre wird die Aufgabe zudem durch eine Füllmaschine zum Füllen von Packungen mit fließfähigen Produkten mit einer endlos umlaufenden Transporteinrichtung zum Transportieren der Packungen durch die Füllmaschine gelöst, wobei die Transporteinrichtung mehrere erfindungsgemäße Zellentraversen umfasst.

Während im Stand der Technik versucht wurde, der unerwünschten Verunreinigung und Kontamination beispielsweise durch häufige oder verbesserte Reinigung oder durch verbesserte Sterilisierungsmittel entgegenzuwirken, schlägt die Erfindung einen anderen Ansatz vor. Gemäß der Erfindung wurde erkannt, dass dadurch, dass bei einer Zellentraverse mit wie beschriebenen Längsstrukturen und Querelementen zur Bildung der Zellen zumindest ein Teil der Querelemente ausschließlich formschlüssig und/oder kraftschlüssig mit den Längsstrukturen verbunden ist, die Ansammlung von Verunreinigungen und die Kontamination von abgefülltem Produkt durch Mikroorganismen verringert oder vermieden werden kann. Es hat sich herausgestellt, dass durch die Verwendung von ausschließlich formschlüssigen und/oder Kraftschlüssigen Verbindungen der Querelemente mit den Längsstrukturen prozesssicher besonders geringe Spalte im Verbindungsbereich erreicht werden können, welche auch bei den im Aseptikbereich einer Füllmaschine herrschenden Bedingungen beständig sind. Hingegen können bei stoffschlüssigen Verbindungen, wie etwa Schweißverbindungen, ein Verzug von bis zu 0,7mm (über die Zellentraversenbreite) und Spalte aufgrund der nicht vollständig zu kontrollierenden Prozesse (beim Schweißen etwa Wärmeeintrag, Wärmeausbreitung oder Schmelzdynamik) und den Bedingungen im Aseptikbereich entstehen. Wie sich gezeigt hat, können derartige Spaltgrößen an Verbindungstellen innerhalb der Zellentraversen dazu führen, dass sich Mikroorganismen hierin ansammeln und damit zu einer Kontamination des aseptischen Bereichs und damit des in die Packung gefüllten Produkts führen. Zudem führt der Verzug dazu, dass es zu Positionierungsungenauigkeiten kommen kann oder jedenfalls das Prozessfenster kleiner wird, und so beispielsweise den Austausch der Kette der Transporteinrichtung früher notwendig werden lässt. Definierte Vorgaben an die Geometrie und die Oberflächengüten (Glattheit) können bei den erfindungsgemäßen Verbindungen hingegen zuverlässiger eingehalten werden, sodass ein Keimwachstum weniger stark initiiert wird. Dadurch, dass zumindest ein Teil der Querelemente ausschließlich formschlüssig und/oder kraftschlüssig mit den Längsstrukturen verbunden ist, kann daher die Gefahr der Ansammlung von Verunreinigungen und der Kontamination von abgefülltem Produkt durch Mikroorganismen verringert werden.

Vorzugsweise sind daher alle Querelemente der Rahmenstruktur der Zellentraverse ausschließlich formschlüssig und/oder kraftschlüssig mit den Längsstrukturen verbunden, um die Gefahr von unerwünschten Verunreinigungen und Kontaminationen weiter zu verringern.

Dass die Zellentraverse für einen Aseptikbereich vorgesehen ist, bedeutet, dass diese dazu ausgebildet ist einen Aseptikbereich zu durchqueren und dass diese auch bei Umgebungsbedingungen, die beispielsweise durch Wasser, Heißluft und/oder Sterilisierungsmittel im Aseptikbereich vorherrschen, dauerhaft beständig ist.

Bevorzugt sind die Längselemente und/oder die Querelemente aus einem Metall, vorzugsweise aus Stahl hergestellt, was bei formschlüssigen und/oder kraftschlüssigen Verbindungen eine wirtschaftliche Herstellung bei hoher Beständigkeit ermöglicht. Aus hygienischen Gründen ist es besonders bevorzugt, wenn die genannten Elemente aus Edelstahl, vorzugsweise einem Edelstahl mit der Werkstoffnummer 1.4301 oder einem höherwertigen Edelstahl dieser Gruppe hergestellt sind. Aus hygienischen Gründen ist es besonders bevorzugt, wenn die genannten Elemente aus Edelstahl, vorzugsweise einem Edelstahl der Sorte 1.4301 (insbesondere mit 17,5 - 19,5% Cr und 0 - 0,1% N) oder einem höherwertigen Edelstahl dieser Gruppe hergestellt sind. Vorzugsweise weist der verwendet Stahl einen PREN-Index (Pitting Resistance Equivalent Number) größer als 15, vorzugsweise größer als 17 auf. Ein Edelstahl der Sorte 1.4301 hat beispielsweise einen PREN-Index von 17,5 - 21,1. Der PREN-Index kann insbesondere gemäß der Formel PREN = 1 x %Cr + 3,3 x %Mo + 16 x %N (w/w) oder alternativ gemäß der Formel PREN = 1 x %Cr + 3,3 x (%Mo + 0,5 x %W) + 16 x %N berechnet werden.

Die erste und die zweite Längsstruktur bilden bevorzugt jeweils eine Längsseite der Rahmenstruktur der Zellentraverse. Die erste und die zweite Längsseite verlaufen dabei bevorzugt parallel. Die Querelemente verlaufen bevorzugt im Wesentlichen senkrecht zu den Längselementen.

Die mehreren in Längsrichtung der Zellentraverse nebeneinander liegenden Zellen müssen dabei nicht unmittelbar nebeneinander liegen. Es ist vielmehr bevorzugt, wenn die Zellen beabstandet in Längsrichtung nebeneinander liegend gebildet werden. Darunter, dass die Zellen zur Aufnahme von zu füllenden oder gefüllten Packungen ausgebildet sind, wird verstanden, dass eine Zelle beispielsweise ganz oder teilweise die Packung aufnehmen kann. Ein oberer Teil der in einer Zelle aufgenommenen Packung ragt bevorzugt über die gebildete Zelle hinaus.

Die Aufnahme oder das Einführen der (leeren) Packungen erfolgt vorzugsweise in einer in Bezug auf die Längsrichtung der Zellentraverse und in Bezug auf die Transportrichtung der Zellentraverse quer (insbesondere senkrecht) liegenden Einführrichtung in die Zellen.

Dadurch, dass die Transporteinrichtung der erfindungsgemäßen Füllmaschine mehrere erfindungsgemäße Zellentraversen umfasst, weist diese eine Vielzahl von Zellen zur Aufnahme der Packungen auf. Dabei weist die endlos umlaufende Transporteinrichtung insbesondere einen Zuführbereich auf, welchem eine Zuführeinrichtung zum Zuführen von zu füllenden Packungen zugeordnet ist. Zudem weist die Transporteinrichtung vorzugsweise einen Abgabebereich auf, welchem eine Abgabeeinrichtung zum Abgeben der Packungen zugeordnet ist. Zwischen dem Zuführbereich und dem Abgabebereich ist dann insbesondere der Aseptikbereich mit Füllbereich vorgesehen. Zwischen dem Abgabebereich und dem Zuführbereich ist vorzugsweise ein Rückführbereich vorgesehen.

Gemäß einer bevorzugten Ausgestaltung der erfindungsgemäßen Zellentraverse ist zumindest ein Teil der Querelemente mittels Nietverbindung mit den Längsstrukturen verbunden. Es hat sich herausgestellt, dass insbesondere durch eine Nietverbindung eine wirtschaftliche Herstellung der Zellentraverse mit geringem Verzug und geringer Spaltbildung erreicht werden kann und im Ergebnis die Ansammlung von Verunreinigungen und die Gefahr der Kontamination von abgefülltem Produkt durch Mikroorganismen verringert werden kann. Beispielsweise wird für jede Verbindungsstelle zwischen Querträger und Längsträger ein Niet verwendet. Ein Niet liegt vorzugsweise koaxial zum jeweiligen Querelement. Die Nietverbindungen können beispielsweise mittels Vollnieten, Blindnieten oder Stanznieten erfolgen. Die Nietverbindung kann formschlüssig und optional kraftschlüssig sein. Durch eine Kaltnietung kann beispielsweise eine ausschließlich formschlüssige Verbindunghergestellt werden, während durch eine Warmnietung beim Abkühlen des Niets durch Schrumpfen zusätzlich ein Kraftschluss erzeugt werden kann.

Gemäß einer bevorzugten Ausgestaltung der erfindungsgemäßen Zellentraverse ist zumindest ein Teil der Querelemente mittels Schraubverbindung mit den Längsstrukturen verbunden. Es hat sich herausgestellt, dass alternativ durch eine Schraubverbindung eine wirtschaftliche Herstellung der Zellentraverse mit geringem Verzug und geringer Spaltbildung erreicht werden kann und im Ergebnis die Ansammlung von Verunreinigungen und die Gefahr der Kontamination von abgefülltem Produkt durch Mikroorganismen verringert werden kann.

Gemäß einer bevorzugten Ausgestaltung der erfindungsgemäßen Zellentraverse ist zumindest ein Teil der Querelemente mittels Steckverbindung mit den Längsstrukturen verbunden. Die Steckverbindung ist beispielsweise eine ausschließlich formschlüssige Verbindung. Beispielsweise wird eine Steckverbindung durch Eingreifen der Querelemente in Schlitze der Längsstruktur und/oder von auf die Längsstruktur aufschiebbaren Hülsen erreicht. Beispielsweise kann die Verbindung dabei durch Auffädeln der Querelemente und der Hülsen auf die Längsstruktur erfolgen, sodass die Querelemente an definierten Positionen formschlüssig mit der Längsstruktur verbunden werden können. Beispielsweise weisen die Hülsen und/oder Längsstreben der Längsstruktur zumindest teilweise umlaufende Nuten oder Schlitze auf, in die die Querelemente eingreifen können. So können dann die Querelemente zusammen mit den Hülsen zunächst auf die Längsstreben der Längsstruktur gesteckt oder geschoben werden. Eine feste Verbindung (Verriegelung) zwischen Querelementen und Längsstruktur ergibt sich dann beispielsweise durch eine endseitige feste Verbindung der ersten seitlichen Längsstruktur mit der zweiten seitlichen Längsstruktur. Alternativ oder zusätzlich kann ein Eindrehen der Längsstreben erfolgen, sodass auch hier zusätzlich eine Art Klips- oder Rastverbindung zwischen den Querelementen und der Längsstruktur hergestellt wird.

Gemäß einer ersten Alternative der erfindungsgemäßen Zellentraverse ist die erste Längsstruktur und/oder die zweite Längsstruktur als im Wesentlichen flächige Seitenwand ausgebildet. Mit einer im Wesentlichen flächigen Seitenwand kann insbesondere bei einer Nietverbindung oder Schraubverbindung eine stabile und prozesssichere Anbindung erfolgen. Die flächige Seitenwand kann beispielsweise als Blech ausgebildet sein. Zur Gewichtsreduktion und Verringerung der Wärmekapazität sind vorzugsweise Aussparungen in der Seitenwand vorgesehen.

Insbesondere im Fall einer Steckverbindung zwischen den Querelementen und den Längsstrukturen, umfasst gemäß einer zweiten Alternative der erfindungsgemäßen Zellentraverse die erste Längsstruktur und/oder die zweite Längsstruktur jeweils zumindest eine, vorzugsweise zumindest zwei Längsstreben. Auf die Längsstreben lassen sich die Querelemente und die Hülsen vorteilhaft aufschieben. Die Längsstreben sind gemäß der zweiten Alternative rohrförmig ausgebildet. Bei mehreren Längsstreben sind die Längsstreben einer Längsstruktur vorzugsweise in Einführrichtung übereinanderliegend angeordnet.

Insbesondere im Fall einer flächigen Längsstruktur ist gemäß der ersten Alternative der erfindungsgemäßen Zellentraverse zumindest ein Teil der Querelemente im Wesentlichen rohrförmig ausgebildet. Im Vergleich zu flächigen Querelementen ermöglichen rohrförmige Querelemente aufgrund der geringeren Blockierung von Reinigungsflüssigkeit insbesondere eine effektive Reinigung der Zellentraverse. Rohrförmige Querelemente sind insbesondere mit einer Nietverbindung prozesssicher anzubinden. Vorzugsweise sind alle Querelemente im Wesentlichen rohrförmig ausgebildet. Bevorzugt haben die rohrförmigen Querelemente zumindest abschnittsweise einen im Wesentlichen runden Querschnitt. Allerding ist auch denkbar, dass ein elliptischer oder eckiger Querschnitt der rohrförmigen Querelemente vorgesehen ist. Insbesondere können die rohrförmigen Querelemente Modifikation aufweisen, welche beispielsweise zur Erzeugung eines definierten Kontaktbereichs mit den Packungen oder zur Anbringung von Einsatzelementen dienen können, wie im Folgenden noch näher beschrieben wird.

Gemäß der zweiten Alternative der erfindungsgemäßen Zellentraverse ist zumindest ein Teil der Querelemente im Wesentlichen flächig ausgebildet. Durch flächige Querelemente lässt sich eine hohe Stabilität insbesondere im Fall von nicht flächigen Längsstrukturen der Rahmenstruktur (beispielsweise bei strebenartigen Längsstrukturen, wie es bei einer Auffädelung der Querelemente vorteilhaft ist) erreichen. Vorzugsweise sind dabei alle Querelemente im Wesentlichen flächig ausgebildet.

Gemäß einer bevorzugten Ausgestaltung der erfindungsgemäßen Zellentraverse weist die Rahmenstruktur der Zellentraverse, insbesondere die Längsstrukturen und/oder die Querelemente, zumindest im Bereich der Zellen in die Zellen hineinragende Kontaktelemente zur Kontaktierung von zu füllenden oder gefüllten Packungen, welche in den Zellen aufzunehmen oder aufgenommen sind, auf. Die Kontaktelemente verhindern eine Kontaktierung der Packung durch die übrige Rahmenstruktur im Bereich der jeweiligen Zelle und verringern damit den Kontaktbereich zwischen Rahmenstruktur und Packung. Hierdurch wird ein zu großer Wärmeübertrag von der erhitzen Packung auf die Rahmenstruktur vermieden, was wiederum die Abkühlung und eine damit verbundene Kondensation (beispielsweise des Sterilisierungsmittels) in der Packung reduziert oder vermeidet. Dies wiederum verringert die Gefahr einer Kontamination der Packungen durch Fremdstoffe. Die Kontaktelemente werden dabei insbesondere durch die Längsstrukturen und/oder Querelemente selbst gebildet. Insbesondere im Falle einer flächigen Längsstruktur können beispielsweise dellenartige Erhebungen in Richtung des Zelleninneren in der flächigen Längsstruktur vorgesehen sein. Insbesondere im Falle von (rohrförmigen) Querelementen können eine oder mehrere umlaufende Verdickungen an den Querelementen vorgesehen sein. Insbesondere im Fall von flächigen Querelementen kann eine flächige, längliche (insbesondere in Einführrichtung verlaufende) Einformung in Richtung des Zelleninneren am Querelement vorgesehen sein.

Gemäß einer bevorzugten Ausgestaltung der erfindungsgemäßen Zellentraverse umfasst die Zellentraverse weiterhin Einsatzelemente, welche jeweils mit der Rahmenstruktur, insbesondere zumindest einer Längsstruktur und/oder zumindest einem, vorzugsweise zwei Querelementen, verbunden oder verbindbar sind und einen Kontaktbereich für gefüllte oder zu füllende Packungen, welche in den Zellen aufzunehmen oder aufgenommen sind, bereitstellen. Durch die Einsatzelemente können die Geometrie und die Eigenschaften (z.B. thermische Eigenschaften) von mit den Packungen in Kontakt zu bringenden Bereichen der Zellentraverse und/oder die Geometrie des durch die Zelle bereitgestellten Aufnahmebereichs (z.B. für unterschiedliche Packungsgrößen) individuell und unabhängig von der Rahmenstruktur angepasst werden. Es hat sich insbesondere als vorteilhaft erwiesen, wenn die Einsatzelemente jeweils zwei Verbindungsbereiche zur Verbindung mit der Rahmenstruktur aufweisen. Beispielsweise weist ein jeweiliges Einsatzelement zwei Verbindungsbereiche zur Verbindung mit einer Längsstruktur auf. Beispielsweise weist ein jeweiliges Einsatzelement zwei Verbindungsbereiche zur Verbindung mit zwei Querelementen auf.

Beispielsweist wird der Kontaktbereich zwischen Packung und Zellentraverse ausschließlich durch die Einsatzelemente bereitgestellt. Es erfolgt also vorzugsweise kein weiterer Kontakt von Packung und Rahmenstruktur.

Die Einsatzelemente sind beispielsweise länglich ausgebildet. Im eingesetzten Zustand verlaufen diese vorzugsweise quer (insbesondere senkrecht) zur Längsrichtung der Rahmenstruktur (das heißt insbesondere in Einführrichtung).

Gemäß einer bevorzugten Ausgestaltung der erfindungsgemäßen Zellentraverse ist zumindest ein Teil der Einsatzelemente (vorzugsweise alle Einsatzelemente) lösbar mit der Rahmenstruktur der Zellentraverse verbunden oder verbindbar, vorzugsweise werkzeuglos verbindbar, insbesondere mittels einer Klipsverbindung, einer Schraubverbindung und/oder einer insbesondere verriegelbaren Steckverbindung. Durch die Lösbarkeit wird ein Austauschen oder Wechseln der Einsatzelemente mit geringem Aufwand möglich. Dabei hat sich gezeigt, dass derartige Verbindungen ausreichend beständig für die in der Füllmaschine vorherrschenden Umgebungsbedingungen sind und die Ablagerung von Verschmutzungen nicht negativ beeinflussen.

Die Einsatzelemente sind vorzugsweise ohne weitere Hilfsmittel (wie beispielsweise bei einer Klipsverbindung) mit der Rahmenstruktur der Zellentraverse verbunden oder verbindbar. Unter Klipsverbindung wird insbesondere eine rastende Verbindung zwischen dem Einsatzelement und der Rahmenstruktur der Zellentraverse verstanden, die unter Überwindung einer gewissen Montagekraft hergestellt wird. Im Fall einer Klipsverbindung hat ein hiermit verbindbares Einsatzelement beispielsweise zumindest eine, vorzugsweise zumindest zwei Rastnuten, insbesondere zur Verbindung mit jeweiligen Querelementen. Ein Querelement kann hierzu an entsprechender Stelle beispielsweise eine umlaufende Nut aufweisen, sodass das Querelement nur im Bereich der Nut den zur Verbindung mit der Rastnut des Einsatzelementes passenden Durchmesser aufweist.

Beispielsweise ist ein Teil der Einsatzelemente mittels Klipsverbindung (insbesondere mit den Querelementen) und ein Teil der Einsatzelemente mittels Schraubverbindung (insbesondere mit der Längsstruktur) verbunden oder verbindbar.

Gemäß einer bevorzugten Ausgestaltung der erfindungsgemäßen Zellentraverse sind die Einsatzelemente aus einem Kunststoff, insbesondere aus einem auf Polyethylenterephthalat basierenden Kunststoff, hergestellt oder weisen einen solchen Kunststoff auf. Durch Einsatzelemente aus Kunststoff kann die Rahmenstruktur beispielsweise aus Metall hergestellt werden, während durch die geringere Wärmeleitfähigkeit des Kunststoffs trotzdem ein geringer Wärmeübertrag auf die Zellentraverse erreicht wird. Beispielsweise umfasst der Kunststoff einen homogen verteilten eingebauten Festschmierstoff, was einen sehr hohen Verschleißwiderstand und einen niedrigeren Gleitreibungskoeffizienten ermöglicht.

Allerdings sind alternativ auch aus Metall, insbesondere Stahl, hergestellte Einsatzelemente möglich, welche eine besonders geringe Gefahr des Bruchs beim Austauschen oder im Betrieb ermöglichen.

Weisen gemäß einer bevorzugten Ausgestaltung der erfindungsgemäßen Zellentraverse die Einsatzelemente zur Bereitstellung der Kontaktfläche zumindest einen federnden Abschnitt auf, kann ein sicherer Sitz von Packungen erreicht werden, ohne diese zu beschädigen. Ein federnder Abschnitt ist insbesondere bei einem aus Kunststoff hergestellten Einsatzelement aufgrund der Elastizität von Kunststoff möglich. Beispielsweise wird das Einsatzelement zur Herstellung des federnden Abschnitts lokal verdünnt ausgebildet und/oder weist einen einseitig freischwingenden Arm auf.

Um sowohl die Gefahr der Kondensation zu verringern und gleichzeitig eine geringe Bruchgefahr der Einsatzelemente zu erreichen, weisen die Einsatzelemente gemäß einer bevorzugten Ausgestaltung der erfindungsgemäßen Zellentraverse eine Metallverstärkung auf. Beispielsweise sind hierzu innere Metalleinsätze in den aus Kunststoff hergestellten Einsatzelementen vorgesehen.

Gemäß einer bevorzugten Ausgestaltung der erfindungsgemäßen Zellentraverse weist die Zellentraverse eine oder mehrere Strömungsleitstrukturen auf, welche jeweils zwischen zwei benachbarten Zellen anordenbar oder angeordnet sind. Die Strömungsleitstrukturen dienen der Lenkung von Gasströmen innerhalb des Aseptikbereichs der Füllmaschine. Vorzugsweise wird der Aseptikbereich mit einem Gas (beispielsweise Sterilluft) dauerhaft gespült, sodass im Aseptikbereich ein nach unten gerichteter Gasstrom vorgesehen ist, um eventuelle Verunreinigungen tendenziell nach unten zu führen. Die Strömungsleitstrukturen sind dabei insbesondere derart vorgesehen, dass sie den Strömungswiderstand im Bereich zwischen benachbarten Zellen vergrößern. Dadurch erfolgt ein höherer Gasstrom im Bereich der Zellen, in deren Bereich die Packungen befüllt werden und eine Kontamination vermieden werden soll. Beispielsweise bilden die Strömungsleitstrukturen einen sich in Einführrichtung zulaufenden Trichter. Beispielsweise umfasst eine Strömungsleitstruktur ein oder mehrere Leitbleche.

Gemäß einer bevorzugten Ausgestaltung der erfindungsgemäßen Zellentraverse sind die eine oder mehreren Strömungsleitstrukturen einstückig mit der Rahmenstruktur, insbesondere der ersten und/oder zweiten seitlichen Längsstruktur ausgebildet. Beispielsweise sind die Strömungsleitstrukturen durch nach innen umgeklappte Bereiche der Längsstruktur ausgebildet.

Gemäß einer bevorzugten Ausgestaltung der erfindungsgemäßen Zellentraverse sind die eine oder mehrere Strömungsleitstrukturen lösbar mit der Rahmenstruktur der Zellentraverse, insbesondere zumindest einer Längsstruktur und/oder zumindest einem Querelement, verbunden oder verbindbar. Auf diese Weise lassen sich die Strömungsleitstrukturen austauschen und aus von der Rahmenstruktur abweichenden Materialien, insbesondere Kunststoff, herstellen. Insbesondere lassen sich die Strömungsleitstrukturen in diesem Fall wie in Zusammenhang mit den Einsatzelementen beschrieben mit der Rahmenstruktur verbinden. Insbesondere können die Strömungsleitstrukturen beispielsweise mittels einer Schraubverbindung oder Klipsverbindung mit der Rahmenstruktur (etwa mit den Längsstrukturen oder den Querelementen) verbindbar oder verbunden sein.

Weitere vorteilhafte beispielhafte Ausgestaltungen der Erfindung sind der folgenden detaillierten Beschreibung beispielhafter Ausführungsformen der vorliegenden Erfindung, insbesondere in Verbindung mit den Figuren, zu entnehmen.

In der Zeichnung zeigen
- Fig. 1a ,b: perspektivische Darstellungen eines ersten Ausführungsbeispiels einer erfindungsgemäßen Zellentraverse;
- Fig. 2a, b: perspektivische Darstellungen eines zweiten Ausführungsbeispiels einer erfindungsgemäßen Zellentraverse;
- Fig. 3a, b: perspektivische Darstellungen eines dritten Ausführungsbeispiels einer erfindungsgemäßen Zellentraverse;
- Fig. 4a, b: schematische Darstellungen zwei weiterer Ausführungsbeispiele von Einsatzelementen; und
- Fig. 5: ein Ausführungsbeispiel einer erfindungsgemäßen Füllmaschine.

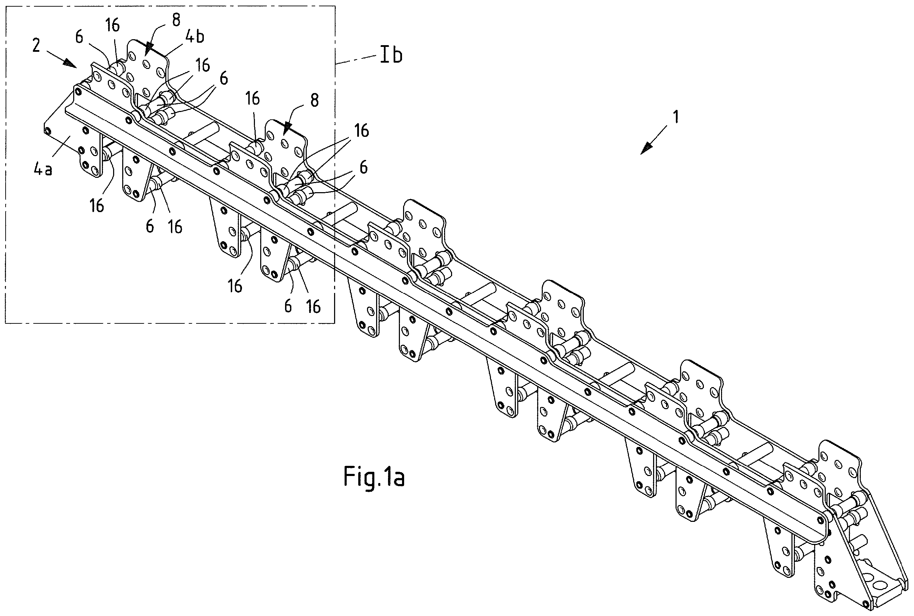

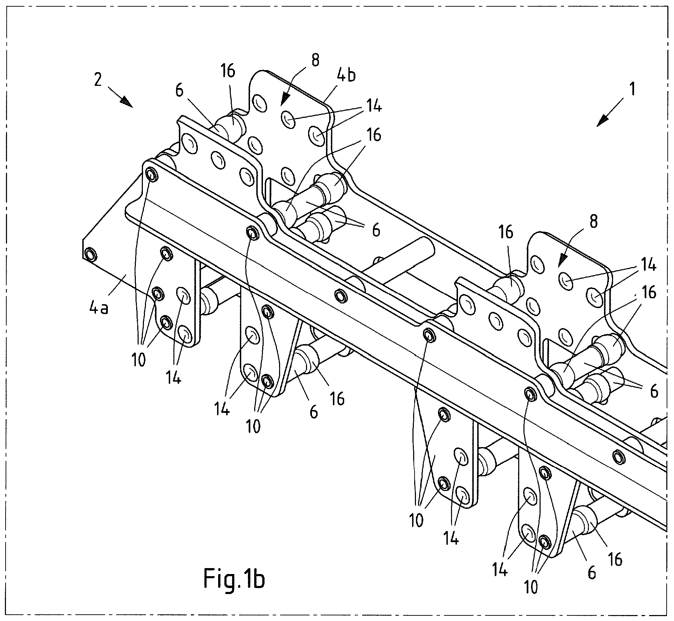

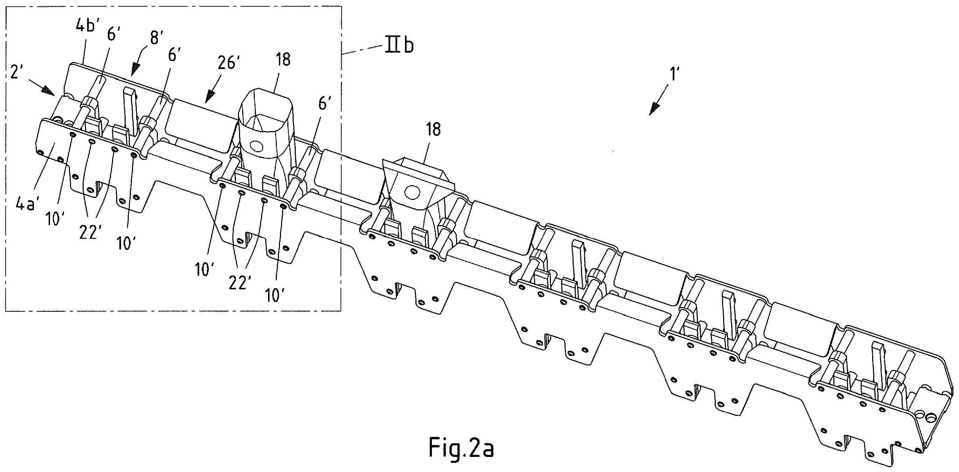

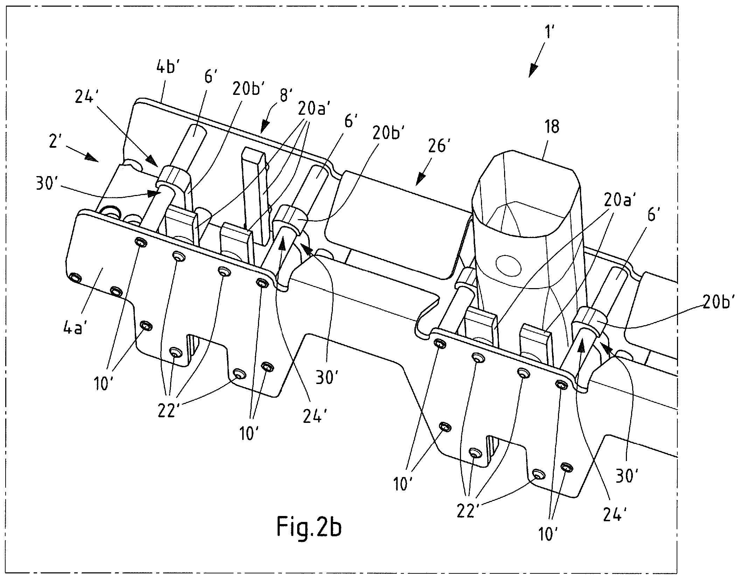

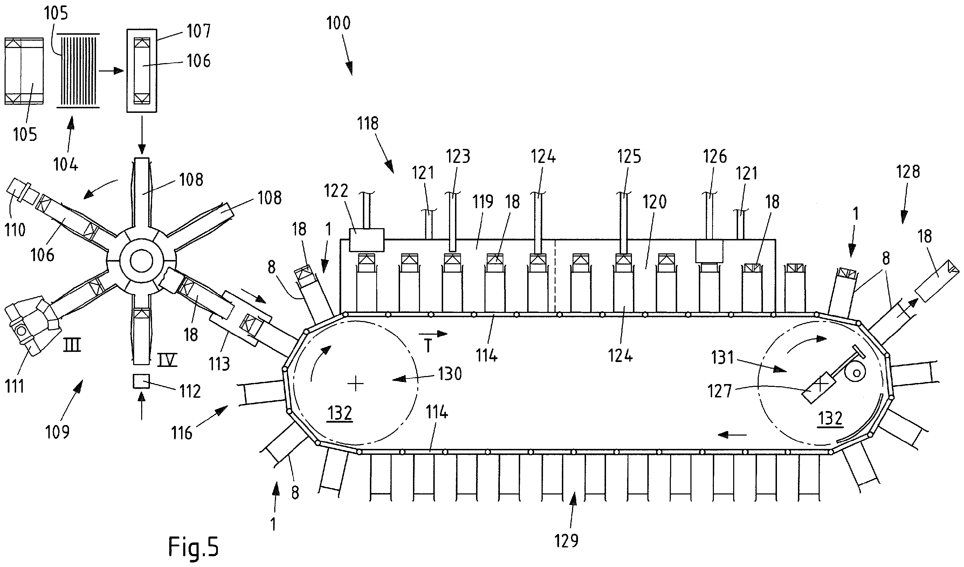

Fig. 1a, b zeigen perspektivische Darstellungen eines ersten Ausführungsbeispiels einer erfindungsgemäßen Zellentraverse 1 von schräg oben mit einem vergrößerten Ausschnitt in Fig. 1b. Die Zellentraverse 1 ist für einen Aseptikbereich einer Füllmaschine (wie sie beispielhaft in Fig. 5 dargestellt ist) zum Füllen von Packungen mit fließfähigen Produkten geeignet. Die Zellentraverse 1 umfasst eine Rahmenstruktur 2 mit einer ersten seitlichen Längsstruktur 4a und einer der ersten Längsstruktur 4a gegenüberliegenden zweiten seitlichen Längsstruktur 4b. Zwischen der ersten und der zweiten Längsstruktur 4a, 4b sind mehrere die Längsstrukturen 4a, 4b verbindende Querelemente 6 angeordnet. Zwischen den Längsstrukturen 4a, 4b und den Querelementen 6 werden mehrere in Längsrichtung der Zellentraverse 1 nebeneinander liegende, aber beabstandete Zellen 8 zur Aufnahme von zu füllenden oder gefüllten Packungen (siehe Fig. 2 oder Fig. 5) gebildet. Die Querelemente 6 sind dabei mittels Nietverbindung 10, das heißt ausschließlich formschlüssig (und optional kraftschlüssig) jeweils beidseitig mit den Längsstrukturen 4a, 4b verbunden. Ebenfalls wäre jedoch auch eine Schraubverbindung anstelle der Nietverbindung 10 für einen Teil oder alle Querelemente 6 möglich.

Die erste Längsstruktur 4a und die zweite Längsstruktur 4b sind als im Wesentlichen flächige Seitenwände ausgebildet und weisen Aussparungen 12 zur Gewichtsreduktion auf. Die Querelemente 6 sind im Wesentlichen rohrförmig ausgebildet.

Die Längsstrukturen 4a, 4b und die Querelemente 6 der Rahmenstruktur 2 der Zellentraverse 1 weisen im Bereich der Zellen 8 in die Zellen 8 hineinragende Kontaktelemente 14, 16 zur Kontaktierung von zu füllenden oder gefüllten Packungen 18 (siehe Fig. 2 oder Fig. 5) auf. Die flächigen Längsstrukturen 4a, 4b weisen dellenartige Erhebungen 14 in Richtung des Zelleninneren auf, während die rohrförmigen Querelemente 6 jeweils eine oder zwei umlaufende Verdickungen 16 aufweisen, um Kontaktbereiche für die Packungen 18 bereitzustellen.

Weiterhin kann die Zellentraverse 1 eine oder mehrere Strömungsleitstrukturen (hier nicht dargestellt) aufweisen, welche jeweils zwischen zwei benachbarten Zellen 8 anordenbar sind. Diese können beispielsweise mittels einer lösbaren Verbindung, wie beispielsweise einer Klipsverbindung an den Querelementen 8 befestigt werden. Ebenfalls wäre jedoch eine integrale Ausbildung beispielsweise mit den Längsstrukturen 4a, 4b (wie beispielsweise in den Fig. 2a - d gezeigt) denkbar.

Die Fig. 2a,b zeigen verschiedene perspektivische Darstellungen eines zweiten Ausführungsbeispiels einer erfindungsgemäßen Zellentraverse 1' von schräg oben, mit einem vergrößerten Ausschntt in Fig. 2b. Ähnlich zur Zellentraverse 1 umfasst auch die Zellentraverse 1' eine Rahmenstruktur 2' mit einer ersten seitlichen flächigen Längsstruktur 4a' und einer zweiten seitlichen flächigen Längsstruktur 4b', zwischen denen mehrere die Längsstrukturen 4a', 4b' verbindende Querelemente 6' angeordnet sind, sodass zwischen den Längsstrukturen 4a', 4b' und den Querelementen 6' mehrere in Längsrichtung der Zellentraverse 1' nebeneinander liegende, aber beabstandete Zellen 8' zur Aufnahme von zu füllenden oder gefüllten Packungen 18 gebildet sind. Die Packungen 18 sind hier zu Illustrationszwecken einmal als oben offene Packung und einmal als bereits versiegelte Packung in Zellen 8' der Zellentraverse 1' eingesetzt dargestellt. Die Querelemente 6' sind wiederum im Wesentlichen rohrförmig ausgebildet. Die Querelemente 6' sind dabei jeweils wiederum mittels Nietverbindung 10' beidseitig mit den Längsstrukturen 4a', 4b' verbunden.

Im Unterschied zu der Zellentraverse 1 aus Fig. 1 weisen die Längsstrukturen 4a', 4b' und die Querelemente 6' der Rahmenstruktur 2' selbst jedoch keine integralen Kontaktelemente auf.

Vielmehr weist die Zellentraverse zusätzlich zur Rahmenstruktur Einsatzelemente 20a', 20b' auf, welche jeweils mit der Rahmenstruktur 2' lösbar verbunden sind. Die Einsatzelemente 20a' sind dabei jeweils mit einer Längsstruktur 4a', 4b' verbunden, während die Einsatzelemente 20b' jeweils mit zwei Querelementen 6' verbunden sind. Hierdurch wird ein Kontaktbereich 30' für gefüllte oder zu füllende Packungen, welche in den Zellen aufzunehmen oder aufgenommen sind, bereitgestellt.

Die lösbare Verbindung wird bei den Einsatzelementen 20a' mittels einer Schraubverbindung 22' an zwei Stellen der Einsatzelemente 20a' mit der jeweiligen Längsstruktur 4a' bzw. 4b'erreicht, während die lösbare Verbindung bei den Einsatzelementen 20b' mittels einer Klipsverbindung 24' an zwei Stellen der Einsatzelemente 20b' mit einem jeweiligen Querelement 6' erreicht wird. Die Einsatzelemente 20b' weisen hierzu Rastnuten auf, welch die Querelemente 6' teilweise umgreifen können. Die Querelemente 6' weisen in diesem Fall im Verbindungsbereich mit den Einsatzelementen 20b' ebenfalls umlaufende Nuten auf, in die die Rastnuten der Einsatzelemente 20b' eingreifen können. Die Einsatzelemente20a' und 20b' sind hier im Wesentlichen länglich ausgebildet und aus einem Kunststoff, insbesondere aus einem auf Polyethylenterephthalat basierenden Kunststoff, hergestellt. Es ist ebenfalls möglich, dass eine Metallverstärkung innerhalb der Einsatzelemente 20a', 20b' vorgesehen wird.

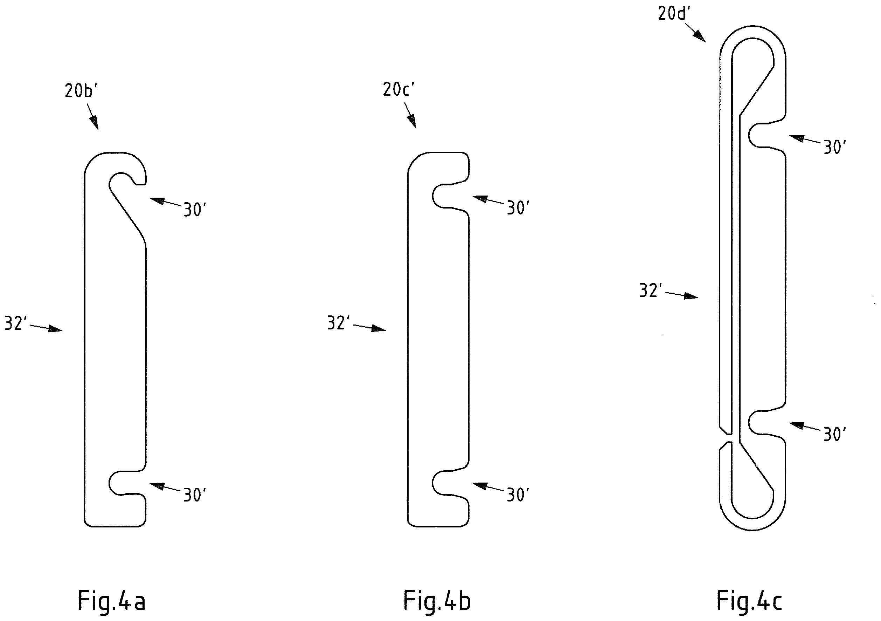

Weitere alternative Ausführungsbeispiele von Einsatzelementen, welche mit einer lösbaren Klipsverbindung mit der Rahmenstruktur (insbesondere den Querelementen) verbunden werden können, sind in Fig. 4 dargestellt.

Weiterhin weist die Zellentraverse 1' mehrere Strömungsleitstrukturen 26' auf, welche jeweils zwischen zwei benachbarten Zellen 8' angeordnet sind. In diesem Fall umfassen die Strömungsleitstrukturen 26' jeweils zwei Leitbleche, welche als umgeklappte Bereiche einstückig mit der ersten bzw. zweiten seitlichen Längsstruktur 4a', 4b' der Rahmenstruktur 2' ausgebildet sind.

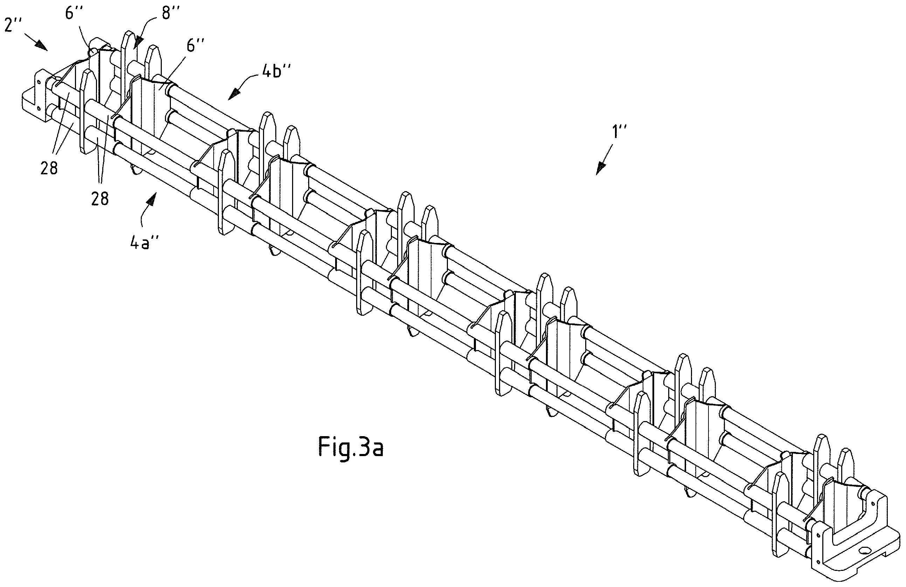

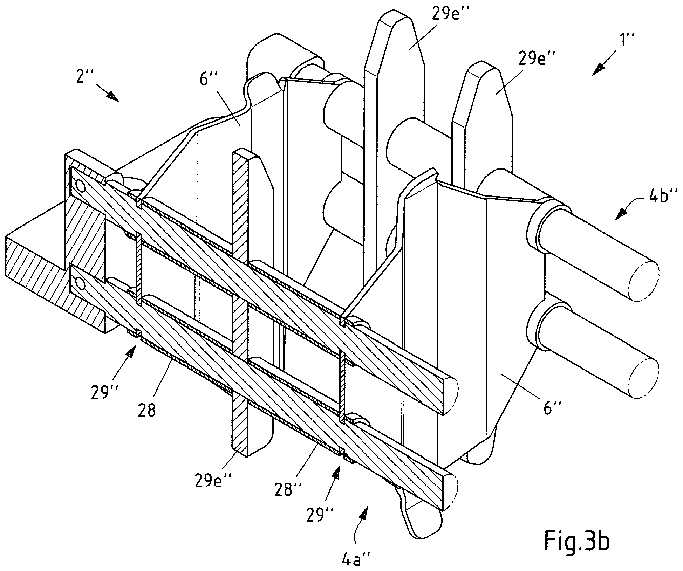

Die Fig. 3a, b zeigen perspektivische Darstellungen eines dritten Ausführungsbeispiels einer erfindungsgemäßen Zellentraverse 1" mit einer vergrößerten Ansicht im Teilquerschnitt in Fig. 3b. Ähnlich zu den Zellentraversen 1, 1' umfasst auch die Zellentraverse 1" eine Rahmenstruktur 2" mit einer ersten seitlichen Längsstruktur 4a" und einer zweiten seitlichen Längsstruktur 4b", zwischen denen mehrere die Längsstrukturen 4a", 4b" verbindende Querelemente 6' angeordnet sind, sodass zwischen den Längsstrukturen 4a", 4b" und den Querelementen 6" mehrere in Längsrichtung der Zellentraverse 1" nebeneinander liegende, beabstandete Zellen 8" zur Aufnahme von zu füllenden oder gefüllten Packungen gebildet sind.

Im Unterschied zu den Zellentraversen 1, 1' umfasst bei der Zellentraverse 1" die erste Längsstruktur 4a" und die zweite Längsstruktur 4b" jeweils zwei Längsstreben. Ebenfalls abweichend von den Zellentraversen 1, 1' sind die Querelemente 6" hier im Wesentlichen flächig ausgebildet. Die Querelemente 6" weisen hier jeweils eine flächige, längliche, in Einführrichtung verlaufende Einformung auf, welche den Kontaktbereich mit der eingeführten Packung definiert. Die Querelemente 6" sind dabei mittels Steckverbindung mit den Längsstrukturen 4a", 4b" verbunden. Die Steckverbindung wird hier durch Eingreifen der Querelemente 6" in Schlitze in den auf die Längsstreben der Längsstruktur aufschiebbaren Hülsen 28" und durch Eingreifen in die Nuten 29" der Längsstreben der Längsstruktur 4a", 4b" erreicht.

Die Führung und Kontaktierung der Packungen 18 im Bereich der Längsstruktur 4a", 4b" erfolgt durch die Einsatzelemente 20e" und stellen einen Kontaktbereich für gefüllte oder zu füllende Packungen 18, welche in den Zellen 8" aufzunehmen oder aufgenommen sind, bereit. Die Einsatzelemente 20e" sind hier durch eine Steckverbindung mit der Längsstruktur 4a", 4b" realisiert, indem die Einsatzelemente 20e" in Längsrichtung jeweils zwischen zwei Hülsen 28" eingeklemmt (und damit verriegelt) sind.

Fig. 4 zeigt nun in schematischen Schnittdarstellungen zwei weitere Ausführungsbeispiele von Einsatzelementen 20c' und 20d', welche insbesondere wie die in Fig. 2 gezeigten Einsatzelemente 20b' verwendet werden können. Die Einsatzelemente 20c' und 20d' weisen jeweils zwei Rastnuten 30' auf, um eine Klipsverbindung 24' mit entsprechenden Querelementen 6' herstellen zu können. Wie die Einsatzelemente 20a', 20b' stellen die Einsatzelemente 20c', 20d' einen Kontaktbereich 32' für gefüllte oder zu füllende Packungen, welche in den Zellen aufzunehmen oder aufgenommen sind, bereit.

Das Einsatzelement 20d' weist zur Bereitstellung des Kontaktbereichs 32' zwei federnde Abschnitte auf, welche jeweils lokal verdünnt ausgebildet sind und einen einseitig freischwingenden Arm aufweisen.

Fig. 5 zeigt nun ein Ausführungsbeispiel einer erfindungsgemäßen Füllmaschine 100 zum Füllen von Packungen 18 mit fließfähigen Produkten mit einer endlos umlaufenden Transporteinrichtung 114 zum Transportieren der Packungen 18 durch die Füllmaschine 100. Die Transporteinrichtung 114 umfasst dabei mehrere in Transportrichtung T hintereinander angeordnete beispielhafte erfindungsgemäße Zellentraversen 1, welche jedoch auch die Zellentraversen 1' oder 1" sein können. Die Zellentraversen 1 sind derart montiert, dass die Längsrichtung der Zellentraversen 1 senkrecht zur Transportrichtung T der Zellentraversen 1 verläuft. Aufgrund der seitlichen Ansicht ist daher pro Zellentraverse 1 nur eine der durch die Zellentraversen 1 bereitgestellten Zellen 8 in Seitenansicht sichtbar.

Die Füllmaschine 1 umfasst in diesem Falle weiterhin eine Formvorrichtung 103 zum Formen von den zu füllenden Packungen 18. Grundsätzlich ist es aber auch möglich, dass der Füllmaschine 100 bereits zu füllende einseitig offene Packungen zugeführt werden. Die dargestellte Füllmaschine 100 weist durch die mehreren Zellen 8 einer Zellentraverse 1 eine Reihe von parallelen Bearbeitungslinien auf, von denen in der Fig. 5 aber lediglich eine Bearbeitungslinie dargestellt ist. Jeder Bearbeitungslinie ist ein Bündel 104 von Packungsrohlingen 105 zugeordnet, deren Längsränder aneinander gesiegelt sind und so beidseitig offene Packungsmäntel 106 bilden. Durch eine Übergabeeinrichtung 107 werden die Packungsmäntel 106 aufgefaltet und auf einen Dorn 108 eines Dornrads 109 geschoben.

Das dargestellte Dornrad 109 wird zyklisch, also schrittweise, gedreht. Dabei werden die Packungsmäntel 106 in unterschiedlichen Stellungen bearbeitet. Zunächst wird ein Rand mit einer Heizeinheit 110 mit Heißluft erwärmt und anschließend in einer Vorfaltung 111 vorgefaltet, um anschließend den vorgefalteten Rand mit einer Presse 112 zu einem Boden zu siegeln. Es wird so eine einseitig offene und an einem Ende dicht verschlossene Packung 18 erhalten, die von einer Zuführeinrichtung 113 an die endlos im Kreis geführte Transporteinrichtung 114 übergeben wird. Das Zuführen kann dabei durch einfaches Abstreifen der Packung 18 vom Dorn 108 und Einschieben in eine Zelle 8 einer Zellentraverse 1 erfolgen. Dabei werden die Packungen 18 nacheinander in separate, im Kreis geführte Zellen 8 der Zellentraversen 1 der Transporteinrichtung 114 eingeführt, die sich jeweils im Zuführbereich 116 der Transporteinrichtung 114 befinden. Es handelt es sich bei der Transporteinrichtung 114 somit um eine endlos umlaufende Zellenkette.

Die Packung 18 wird durch die Transporteinrichtung 114 durch einen Aseptikbereich 118 transportiert, der nacheinander in eine Sterilisationszone 119 und eine Füll- und Siegelzone 120 unterteilt ist. Der Transport der Packungen 18 muss nicht geradlinig erfolgen, sondern kann auch in wenigstens einem Bogen oder gar im Kreis erfolgen, je nachdem ob es sich bei der Füllmaschine 100 um einen sogenannten Längsläufer oder einen Rundläufer handelt. Der Aseptikbereich in der Aseptikkammer 118 wird über entsprechende Sterilluftanschlüsse 121 mit Sterilluft gespült, um das Eindringen von Keimen zu verhindern.

In die oben offenen Packungen 18 wird zunächst durch eine Vorwärmeinrichtung 122 sterile Heißluft eingeblasen, um die Packungen 18 vorzuwärmen. Anschließend wird in einer Sterilisiereinrichtung 123 ein Sterilisierungsmittel in die Packungen eingeblasen, das in den vorgewärmten Packungen 18 stärker reagiert und weniger kondensiert. Als Sterilisierungsmittel wird beispielsweise Wasserstoffperoxid verwendet. Das Wasserstoffperoxid kann zusammen mit Dampf und/oder Luft in die einseitig offene Packung 18 eingeleitet werden. Nach dem Sterilisieren der Packungen 18 wird das Innere der Packungen 18 in einer Trocknungseinrichtung 124 durch Beaufschlagen mit Trocknungsluft getrocknet. Die Trocknungsluft ist dabei vorzugsweise heiß und steril.

Nach dem Übergang von der Sterilisationszone 119 in die Füll- und Siegelzone 120 werden die einseitig offenen Packungen 18 unter einer Fülleinrichtung 125 positioniert und mit einem Lebensmittel gefüllt. Die gefüllten Packungen 18 werden sodann mit einer Verschließeinrichtung 126 durch Falten des oberen offenen Bereichs der Packung 18 und Siegeln desselben verschlossen. Die verschlossenen Packungen 18 werden anschließend über eine Abgabeeinrichtung 127 in einem Abgabebereich 128 der Transporteinrichtung 114 entgegen der Einführrichtung aus den Zellen 8 der Transporteinrichtung 114 abgegeben und sodann bedarfsweise weiter bearbeitet. Die nun leeren Zellen 8 der Transporteinrichtung 114 werden über einen Rückführbereich 129 der Transporteinrichtung 114 weiter in Richtung des Dornrads 109 bewegt, um dort weitere Packungen 18 aufzunehmen. Um die Zellen 8 endlos hin und her transportieren zu können, sind dem Zuführbereich 116 und dem Abgabebereich 128 Umlenkbereiche 130, 131 zugeordnet, in denen bei der Füllmaschine 100 sogenannte Umlenkrollen 132 vorgesehen sind.

## Patentansprüche

1. Zellentraverse für einen Aseptikbereich einer Füllmaschine (100) zum Füllen von Packungen (18) mit fließfähigen Produkten, wobei die Zellentraverse (1, 1', 1") eine Rahmenstruktur (2, 2', 2") mit
- zumindest einer ersten seitlichen Längsstruktur (4a, 4a', 4a"),
- zumindest einer der ersten Längsstruktur (4a, 4a', 4a") gegenüberliegenden zweiten seitlichen Längsstruktur (4b, 4b', 4b"), und
- mehreren zwischen der ersten und der zweiten Längsstruktur (4a, 4a', 4a", 4b, 4b', 4b") angeordneten und die Längsstrukturen'verbindenden Querelementen (6, 6', 6"),
umfasst, wobei zwischen den Längsstrukturen (4a, 4a', 4a", 4b, 4b', 4b") und den Querelementen (6, 6', 6") mehrere in Längsrichtung der Zellentraverse (1, 1', 1") nebeneinander liegende Zellen (8, 8', 8") zur Aufnahme von zu füllenden oder gefüllten Packungen (18) gebildet werden,
**dadurch gekennzeichnet, dass**
die Zellen (8, 8', 8") jeweils durch die Längsstrukturen (4a, 4a', 4a", 4b, 4b', 4b") und Querelemente (6, 6', 6") gebildet werden, wobei entweder
- die erste Längsstruktur und/oder die zweite Längsstruktur (4a, 4a', 4a", 4b, 4b', 4b") als im Wesentlichen flächige Seitenwand ausgebildet ist und zumindest ein Teil der Querelemente (6, 6', 6") im Wesentlichen rohrförmig ausgebildet ist,
oder
- die erste Längsstruktur und/oder die zweite Längsstruktur (4a, 4a', 4a", 4b, 4b', 4b") jeweils zumindest eine, vorzugsweise zumindest zwei rohrförmige Längsstreben umfasst und zumindest ein Teil der Querelemente (6,6',6") im Wesentlichen flächig ausgebildet ist,
und wobei zumindest ein Teil der Querelemente (6, 6' 6") ausschließlich formschlüssig und/oder kraftschlüssig mit den Längsstrukturen (4a, 4a', 4a", 4b, 4b', 4b") verbunden ist.

2. Zellentraverse nach Anspruch 1,
wobei zumindest ein Teil der Querelemente (6, 6', 6") mittels Nietverbindung (10, 10') mit den Längsstrukturen (4a, 4a', 4a", 4b, 4b', 4b") verbunden ist.

3. Zellentraverse nach Anspruch 1 oder 2,
wobei zumindest ein Teil der Querelemente (6, 6', 6") mittels Schraubverbindung mit den Längsstrukturen (4a, 4a', 4a", 4b, 4b', 4b") verbunden ist.

4. Zellentraverse nach einem der Ansprüche 1 bis 3,
wobei zumindest ein Teil der Querelemente (6, 6', 6") mittels Steckverbindung mit den Längsstrukturen (4a, 4a', 4a", 4b, 4b', 4b") verbunden ist.

5. Zellentraverse nach einem der Ansprüche 1 bis 4,
wobei die Rahmenstruktur (2,2',2") der Zellentraverse (1,1',1"), insbesondere die Längsstrukturen (4a, 4a', 4a", 4b, 4b', 4b") und/oder die Querelemente (6,6', 6"), zumindest im Bereich der Zellen (8, 8', 8") in die Zellen (8, 8', 8") hineinragende Kontaktelemente (14,16) zur Kontaktierung von zu füllenden oder gefüllten Packungen (18), welche in den Zellen (8, 8', 8") aufzunehmen oder aufgenommen sind, aufweist.

6. Zellentraverse nach einem der Ansprüche 1 bis 5,
wobei die Zellentraverse (1, 1', 1") weiterhin Einsatzelemente (20a', 20b', 20c', 20d', 20e") umfasst, welche jeweils mit der Rahmenstruktur (2, 2', 2"), insbesondere zumindest einer Längsstruktur(4a, 4a', 4a", 4b, 4b', 4b") und/oder zumindest einem, vorzugsweise zwei Querelementen (6, 6', 6"), verbunden oder verbindbar sind und einen Kontaktbereich (32') für gefüllte oder zu füllende Packungen (18), welche in den Zellen (8, 8', 8") aufzunehmen oder aufgenommen sind, bereitstellen.

7. Zellentraverse nach Anspruch 6,
wobei zumindest ein Teil der Einsatzelemente (20a', 20b', 20c', 20d', 20e") lösbar mit der Rahmenstruktur der Zellentraverse verbunden oder verbindbar ist, vorzugsweise werkzeuglos verbindbar, insbesondere mittels einer Klipsverbindung (24'), einer Schraubverbindung (22') und/oder einer insbesondere verriegelbaren Steckverbindung.

8. Zellentraverse nach Anspruch 6 oder 7,
wobei die Einsatzelemente (20a', 20b', 20c', 20d', 20e") aus einem Kunststoff, insbesondere aus einem auf Polyethylenterephthalat basierenden Kunststoff, hergestellt sind oder einen solchen Kunststoff aufweisen.

9. Zellentraverse nach einem der Ansprüche 6 bis 8,
wobei die Einsatzelemente (20a', 20b', 20c', 20d', 20e") zur Bereitstellung des Kontaktbereichs (32') zumindest einen federnden Abschnitt aufweisen.

10. Zellentraverse nach einem der Ansprüche 6 bis 9,
wobei die Einsatzelemente (20a', 20b', 20c', 20d', 20e") eine Metallverstärkung aufweisen.

11. Zellentraverse nach einem der Ansprüche 1 bis 10,
wobei die Zellentraverse (1, 1', 1") eine oder mehrere Strömungsleitstrukturen zur Lenkung von Gasströmen innerhalb des Aseptikbereichs der Füllmaschine (26') aufweist, welche jeweils zwischen zwei benachbarten Zellen (8,8',8") anordenbar oder angeordnet sind.

12. Zellentraverse nach Anspruch 11,
wobei die eine oder mehreren Strömungsleitstrukturen (26') einstückig mit der Rahmenstruktur (2, 2', 2"), insbesondere der ersten und/oder zweiten seitlichen Längsstruktur (4a, 4a', 4a", 4b, 4b', 4b") ausgebildet sind.

13. Zellentraverse nach Anspruch 11 oder 12,
wobei die eine oder mehreren Strömungsleitstrukturen (26') lösbar mit der Rahmenstruktur (2, 2', 2") der Zellentraverse (1, 1', 1"), insbesondere zumindest einer Längsstruktur (4a, 4a', 4a", 4b, 4b', 4b") und/oder zumindest einem Querelement (6, 6', 6"), verbunden oder verbindbar sind.

14. Verwendung einer Zellentraverse (1, 1', 1") nach einem der Ansprüche 1 bis 13 für eine Füllmaschine (100) zum Füllen von Packungen (18) mit fließfähigen Produkten.

15. Füllmaschine zum Füllen von Packungen (18) mit fließfähigen Produkten mit einer endlos umlaufenden Transporteinrichtung (114) zum Transportieren der Packungen (18) durch die Füllmaschine (100), wobei die Transporteinrichtung (114) mehrere Zellentraversen (1, 1', 1") nach einem der Ansprüche 1 bis 13 umfasst.

## Claims

1. Cell traverse for an aseptic area of a filling machine (100) for filling packages (18) with flowable products, wherein the cell traverse (1, 1', 1") comprises a frame structure (2, 2', 2") with
- at least one first lateral longitudinal structure (4a, 4a', 4a"),
- at least one second lateral longitudinal structure (4b, 4b', 4b") opposite the first longitudinal structure (4a, 4a', 4a"), and
- a plurality of transverse elements (6, 6', 6") arranged between the first and second longitudinal structure (4a, 4a', 4a", 4b, 4b', 4b") and connecting the longitudinal structures,
wherein a plurality of cells (8, 8', 8") situated adjacent to one another in the longitudinal direction of the cell traverse (1, 1', 1") for receiving filled or to-be-filled packages (18) are formed between the longitudinal structures (4a, 4a', 4a", 4b, 4b', 4b") and the transverse elements (6, 6', 6"),
**characterized in that**
the cells (8, 8', 8") are each formed by the longitudinal structures (4a, 4a', 4a", 4b, 4b") and transverse elements (6, 6', 6"), wherein either
- the first longitudinal structure and/or the second longitudinal structure (4a, 4a', 4a", 4b, 4b', 4b") is formed as a substantially planar side wall and at least part of the transverse elements (6, 6', 6") is formed substantially tubular,
or
- the first longitudinal structure and/or the second longitudinal structure (4a, 4a', 4a", 4b, 4b', 4b") each comprise at least one, preferably at least two tubular longitudinal struts and at least part of the transverse elements (6, 6', 6") is formed substantially planar,
and wherein at least part of the transverse elements (6, 6', 6") is connected to the longitudinal structures (4a, 4a', 4a", 4b, 4b', 4b") exclusively in a positive-locking and/or force-locking manner.

2. Cell traverse according to claim 1,
wherein at least part of the transverse elements (6, 6', 6") is connected to the longitudinal structures (4a, 4a', 4a", 4b, 4b', 4b") by means of a rivet connection (10, 10').

3. Cell traverse according to claim 1 or 2,
wherein at least part of the transverse elements (6, 6', 6") is connected to the longitudinal structures (4a, 4a', 4a", 4b, 4b', 4b") by means of a screw connection.

4. Cell traverse according to one of claims 1 to 3,
wherein at least part of the transverse elements (6, 6', 6") is connected to the longitudinal structures (4a, 4a', 4a", 4b, 4b', 4b") by means of a plug connection.

5. Cell traverse according to one of claims 1 to 4,
wherein the frame structure (2, 2', 2") of the cell traverse (1, 1', 1"), in particular the longitudinal structures (4a, 4a', 4a", 4b, 4b', 4b") and/or the transverse elements (6, 6', 6"), at least in the region of the cells (8, 8', 8") has contact elements (14, 16) protuding into the cells (8, 8', 8") for contacting filled or to-be-filled packages (18) which are received or to-be-received in the cells (8, 8', 8").

6. Cell traverse according to one of claims 1 to 5,
wherein the cell traverse (1, 1', 1") further comprises insert elements (20a', 20b', 20c', 20d', 20e") which are each connected or connectable to the frame structure (2, 2', 2"), in particular at least one longitudinal structure (4a, 4a', 4a", 4b, 4b', 4b") and/or at least one, preferably two transverse elements (6, 6', 6"), and provide a contact area (32') for filled or to-be-filled packages (18) which are received or to-be-received in the cells (8, 8', 8").

7. Cell traverse according to claim 6,
wherein at least part of the insert elements (20a', 20b', 20c', 20d', 20e") is detachably connected or connectable to the frame structure of the cell traverse, preferably connectable without tools, in particular by means of a clip connection (24'), a screw connection (22') and/or a plug connection that is in particular lockable.

8. Cell traverse according to claim 6 or 7,
wherein the insert elements (20a', 20b', 20c', 20d', 20e") are made of a plastic, in particular of a plastic based on polyethylene terephthalate, or comprise such a plastic.

9. Cell traverse according to one of claims 6 to 8,
wherein the insert elements (20a', 20b', 20c', 20d', 20e") comprise at least one resilient portion for providing the contact area (32').

10. Cell traverse according to one of claims 6 to 9,
wherein the insert elements (20a', 20b', 20c', 20d', 20e") comprise a metal reinforcement.

11. Cell traverse according to one of claims 1 to 10,
wherein the cell traverse (1, 1', 1") comprises one or several flow guiding structures for guiding gas flows within the aseptic area of the filling machine (26'), which are each arranged or arrangeable between two adjacent cells (8, 8', 8").

12. Cell traverse according to claim 11,
wherein the one or several flow guiding structures (26') are formed integrally with the frame structure (2, 2', 2"), in particular the first and/or second lateral longitudinal structure (4a, 4a', 4a", 4b, 4b', 4b").

13. Cell traverse according to claim 11 or 12,
wherein the one or several flow guiding structures (26') are detachably connected or connectable to the frame structure (2, 2', 2") of the cell traverse (1, 1', 1"), in particular at least one longitudinal structure (4a, 4a', 4a", 4b, 4b', 4b") and/or at least one transverse element (6, 6', 6").

14. Use of a cell traverse (1, 1', 1") according to one of claims 1 to 13 for a filling machine (100) for filling packages (18) with flowable products.

15. Filling machine for filling packages (18) with flowable products, comprising a continuously circulating transport device (114) for transporting the packages (18) through the filling machine (100), wherein the transport device (114) comprises a plurality of cell traverses (1, 1', 1") according to one of claims 1 to 13.

## Revendications

1. Traverse de cellules pour une zone aseptique d'une machine de remplissage (100) pour remplir des emballages (18) avec des produits fluides, la traverse de cellules (1, 1', 1") comprenant une structure de cadre (2, 2', 2") avec
- au moins une première structure longitudinale latérale (4a, 4a', 4a"),
- au moins une deuxième structure longitudinale latérale (4b, 4b', 4b") opposée à la première structure longitudinale (4a, 4a', 4a"), et
- une pluralité d'éléments transversaux (6, 6', 6") disposés entre la première et la deuxième structure longitudinale (4a, 4a', 4a", 4b, 4b', 4b") et reliant les structures longitudinales,
une pluralité de cellules (8, 8', 8") situées adjacentes les unes aux autres dans la direction longitudinale de la traverse de cellules (1, 1', 1") pour recevoir des emballages (18) à remplir ou remplis étant formées entre les structures longitudinales (4a, 4a', 4a", 4b, 4b', 4b") et les éléments transversaux (6, 6', 6"), **caractérisée en ce que**
les cellules (8, 8', 8") sont formées chacune par les structures longitudinales (4a, 4a', 4a", 4b, 4b") et les éléments transversaux (6, 6', 6"), soit
- la première structure longitudinale et/ou la deuxième structure longitudinale (4a, 4a', 4a", 4b, 4b', 4b") étant formée comme une paroi latérale essentiellement plate et au moins une partie des éléments transversaux (6, 6', 6") étant formée de manière essentiellement tubulaire,
ou
- la première structure longitudinale et/ou la deuxième structure longitudinale (4a, 4a', 4a", 4b, 4b', 4b") comprenant chacune au moins une, de préférence au moins deux entretoises longitudinales tubulaires et au moins une partie des éléments transversaux (6, 6', 6") étant formée de manière essentiellement plate,
et au moins une partie des éléments transversaux (6, 6', 6") étant reliée aux structures longitudinales (4a, 4a', 4a", 4b, 4b', 4b") exclusivement par engagement positif et/ou par force.

2. Traverse de cellules selon la revendication 1,
au moins une partie des éléments transversaux (6, 6', 6") étant reliée aux structures longitudinales (4a, 4a', 4a", 4b, 4b', 4b") au moyen d'une connexion rivetée (10, 10').

3. Traverse de cellules selon la revendication 1 ou 2,
au moins une partie des éléments transversaux (6, 6', 6") étant reliée aux structures longitudinales (4a, 4a', 4a", 4b, 4b', 4b") au moyen d'une connexion par vis.

4. Traverse de cellules selon l'une des revendications 1 à 3,
au moins une partie des éléments transversaux (6, 6', 6") étant reliée aux structures longitudinales (4a, 4a', 4a", 4b, 4b', 4b") au moyen d'une connexion par enfichage.

5. Traverse de cellules selon l'une des revendications 1 à 4,
la structure du cadre (2, 2', 2") de la traverse de cellules (1, 1', 1"), en particulier les structures longitudinales (4a, 4a', 4a", 4b, 4b', 4b") et/ou les éléments transversaux (6, 6', 6"), au moins dans la zone des cellules (8, 8', 8") présente des éléments de contact (14, 16) faisant saillie dans les cellules (8, 8', 8") pour entrer en contact avec des emballages (18) à remplir ou remplis qui doivent être reçus ou sont reçus dans les cellules (8, 8', 8").

6. Traverse de cellules selon l'une des revendications 1 à 5,
la traverse de cellules (1, 1', 1") comprenant en outre des éléments d'insertion (20a', 20b', 20c', 20d', 20e") qui sont reliés chacun à la structure de cadre (2, 2', 2"), en particulier à au moins une structure longitudinale (4a, 4a', 4a", 4b, 4b', 4b") et/ou au moins un, de préférence deux éléments transversaux (6, 6', 6") et fournissent une zone de contact (32') pour des emballages (18) à remplir ou remplis qui doivent être reçus ou sont reçus dans les cellules (8, 8', 8").

7. Traverse de cellules selon la revendication 6,
dans laquelle au moins une partie des éléments d'insertion (20a', 20b', 20c', 20d', 20e") est reliée ou peut être reliée de manière amovible à la structure du cadre de la traverse de cellules, de préférence sans outils, en particulier au moyen d'une connexion par clip (24'), d'une connexion parvis (22') et/ou d'une connexion par enfichage qui est en particulier verrouillable.

8. Traverse de cellules selon la revendication 6 ou 7,
les éléments d'insertion (20a', 20b', 20c', 20d', 20e") étant constitués d'une matière plastique, en particulier d'une matière plastique à base de polyéthylène téréphtalate, ou comprenant une telle matière plastique.

9. Traverse de cellules selon l'une des revendications 6 à 8,
les éléments d'insertion (20a', 20b', 20c', 20d', 20e") comprenant au moins une partie élastique pour fournir la zone de contact (32').

10. Traverse de cellules selon l'une des revendications 6 à 9,
les éléments d'insertion (20a', 20b', 20c', 20d', 20e") comprenant un renfort métallique.

11. Traverse de cellules selon l'une des revendications 1 à 10,
la traverse de cellules (1, 1', 1") comprenant une ou plusieurs structures de guidage de flux pour guider les flux gazeux au sein de la zone aseptique de la machine de remplissage (26'), qui peuvent être disposée ou sont disposée chacune entre deux cellules adjacentes (8, 8', 8").

12. Traverse de cellules selon la revendication 11,
la une ou les plusieurs structures de guidage de flux (26') étant formées d'un seul tenant avec la structure de cadre (2, 2', 2"), en particulier avec la première et/ou la deuxième structure longitudinale latérale (4a, 4a', 4a", 4b, 4b', 4b").

13. Traverse de cellules selon la revendication 11 ou 12,
la une ou les plusieurs structures de guidage de flux (26') étant reliées ou pouvant être reliées de manière amovible à la structure de cadre (2, 2', 2") de la traverse de cellules (1, 1', 1"), en particulier à au moins une structure longitudinale (4a, 4a', 4a", 4b, 4b', 4b") et/ou au moins un élément transversal (6, 6', 6").

14. Utilisation d'une traverse de cellules (1, 1', 1") selon l'une des revendications 1 à 13 pour une machine de remplissage (100) pour remplir des emballages (18) avec des produits fluides.

15. Machine de remplissage pour remplir des emballages (18) avec des produits fluides, comprenant un dispositif de transport (114) à circulation sans fin pour transporter les emballages (18) à travers la machine de remplissage (100), le dispositif de transport (114) comprenant une pluralité de traverses de cellules (1, 1', 1") selon l'une des revendications 1 à 13.
